# EUROPEAN PATENT SPECIFICATION

(11) **EP 4 051 246 B1**
(45) Date of publication and mention of the grant of the patent: **18.03.2026**
(21) Application number: 20881133.1
(22) Date of filing: 27.10.2020
(51) Int. Cl.: A61K 9/20, A61K 31/155, A61K 31/351, A61P 3/10

(54) **BILAYER TABLET FORMULATIONS COMPRISING DAPAGLIFLOZIN AND METFORMIN**
ZWEISCHICHTIGE BRAUSETABLETTENFORMULIERUNGEN MIT DAPAGLIFLOZIN UND METFORMIN
FORMULATIONS DE COMPRIMÉS BICOUCHES COMPRENANT DE LA DAPAGLIFLOZINE ET DE LA METFORMINE

(30) Priority: 31.10.2019 TR 201916829
(43) Date of publication of application: 07.09.2022
(73) Proprietor: Sanovel Ilac Sanayi ve Ticaret A.S., Sisli - Istanbul (TR)
(72) Inventor: TASKIN, Abdullah, 34460 Sar yer/Istanbul (TR); DEDEOGLU, Yavuz, 34460 Sar yer/Istanbul (TR); ULUSOY BOZYEL, Muge, 34460 Sar yer/Istanbul (TR)
(74) Representative: Genç Ilhan, Oznur
(86) International application number: PCT/TR2020/050996
(87) International publication number: WO 2021/086292

(56) References cited:
- WO-A2-2011/060256
- US-A1- 2011 311 594
- US-A1- 2012 041 069
- US-A1- 2017 333 353

## Description

### Field of Invention

The invention relates to formulations comprising dapagliflozin or a pharmaceutically acceptable salt thereof combined with metformin or a pharmaceutically acceptable salt thereof. More particularly, the present invention relates to the bilayer tablet formulation of dapagliflozin and metformin with the desired properties.

### Background of the Invention

Diabetes mellitus, known as diabetes, is a lifelong chronic disease resulting from the pancreas's inability to produce enough insulin or the body's ability to use insulin effectively, and it continues with the reduction of insulin-producing cells. Blood glucose rises with absent or non-functioning insulin hormone. The resulting high blood sugar causes classical symptoms such as polyuria (frequent urination), polydipsia (increased thirst) and polyphagia (increased hunger). Currently, there are three types of diabetes: type 1 diabetes (type 1), type 2 diabetes (type 2) and gestational diabetes. Gestational diabetes is seen in pregnant women who develop high blood sugar levels. Type 1 diabetes is caused by the body's inability to produce insulin, requiring insulin injection. Finally, with type 2 diabetes, the body either resists the effects of insulin or does not produce enough insulin to maintain a normal glucose level. Of these three types of diabetes, type 2 diabetes is the most common type, affecting more than 171 million people worldwide.

Dapagliflozin is a sodium-glucose cotransporter 2 inhibitor (SGLT2). SGLT2 is a carrier responsible for the reabsorption of most of the glucose from the lumen of the renal tubule. SGLT2 is expressed in proximal renal tubules. By inhibiting SGLT2, dapagliflozin reduces the reabsorption of the filtered glucose and lowers the renal threshold for glucose. This improves urinary glucose excretion and blood glucose control. Dapagliflozin, also known as (1S)-1,5-Anhydro-1-C-[4-chloro-3-[(4-ethoxyphenyl)methyl]phenyl]-D-glucitol or (2S,3R,4R,5S,6R)-2-(3-(4-etoxybenzyl)-4-chlorophenyl)-6-hydroxymethyltetrahydro-2H-pyran-3,4,5-triol is represented by the structure of Formula I.

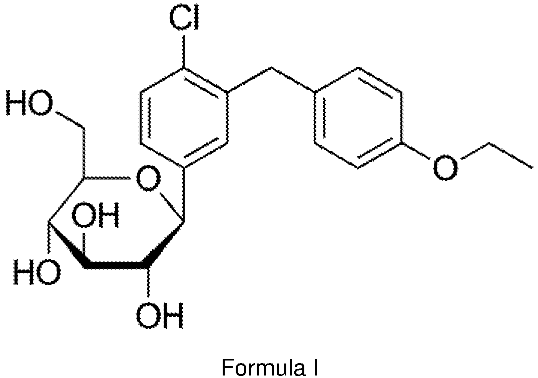

Dapagliflozin was first disclosed in patent US 6515117 (2003, Bristol-Myers Squibb).

Metformin is an antihyperglycemic agent that improves glucose tolerance and lowers both basal and postprandial plasma glucose levels by different mechanisms than other oral antidiabetic agents. Metformin decreases hepatic gluconeogenesis, decreases intestinal absorption of glucose, and improves insulin sensitivity by increasing peripheral glucose uptake and utilization. With metformin therapy, insulin secretion remains unchanged while fasting insulin levels and day-long plasma insulin response may actually decrease. Metformin, also known as N, N-dimethylimidodicarbonimidic diamide or 1,1-Dimethylbiguanide or N, N-dimethyldiguanide or N, N-Dimethylguanylguanidine, is represented by the chemical structure in Formula II.

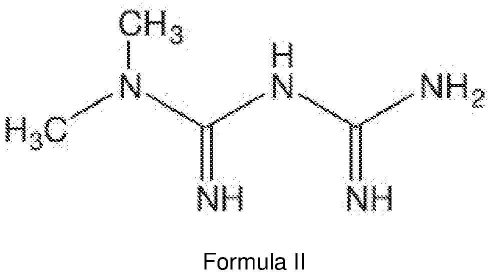

Metformin was first disclosed in patent US 3174901 (1965, Jan Marcel Didier Aron-Samuel).

EP2498758B1 discloses bilayer tablets comprising a first layer, wherein the first layer is extended release metformin and a second layer, wherein the second layer is immediate release dapagliflozin molecule.

EP2498759B1 discloses a process for the preparation of an immediate release formulation comprising a combination of dapagliflozin and metformin.

WO 2017/098481 discloses an effervescent combination comprising metformin with retained carbon dioxide content of at least 90% of the input blend and a different antidiabetic agent.

Unexamined patent TR2012/02948 discloses a tablet form comprising a combination of metformin hydrochloride and dapagliflozin and a core tablet coated with a coating solution containing extended release metformin and dapagliflozin.

The main advantage of bilayer tablet formulations comprising dapagliflozin or a pharmaceutically acceptable salt thereof with metformin or a pharmaceutically acceptable salt thereof compared to other drug forms is the immediate effect of the drug experienced by the target group of patients, which continues throughout the day. Thus, the target patient group does not need to use drugs repeatedly.

The main object of bilayer tablet formulations comprising dapagliflozin or a pharmaceutically acceptable salt thereof with metformin or a pharmaceutically acceptable salt thereof is to increase patient compliance by facilitating patient's life with a decreased number of drugs used by type 2 diabetes patients daily.

In the state of the art, there are tablet configurations with layers that provide immediate and extended releases. Immediate release layers balance the amount of glucose in the blood immediately, while the extended release layers maintain the blood sugar balance over an extended period. But these configurations have undesired properties. Following administration, disintegration and dissolution begin in the immediate and extended release layers and the active substance diffuses into the environment. However, if the immediate release layer does not diffuse fast enough, the polymers that release and control the release in extended release layers creates a diffusion barrier surrounding the tablet. This barrier prevents the immediate release layer, which does not release fast enough, from diffusing and the drug from reaching its desired efficiency. As a result, the desired release profile cannot be achieved. The present formulations require additional time for the onset of action. This is undesirable for the patient. The early onset of the treatment process directly affects the patient's quality of life.

In addition, the solubility of the metformin molecule is high whereas the solubility of the dapagliflozin molecule is low. Achieving the intended release profile is quite difficult.

US 2017/333353 A1 discloses a bilayer tablet formulation wherein an extended-releasee layer contains metformin and an immediate release layer contains dapagliflozin.

US 2012/041069 A1 relates to controlled release compositions which comprises a sustained release layer and an immediate release layer. According to Example-1 of US 2012/041069 A1; metformin is in the sustained release layer; while dapagliflozin is in the immediate release layer.

The selection and proportion of excipients with appropriate release profile for the desired drug configuration, promoting the release from the immediate release layer at the desired rate is of critical importance.

In order to eliminate the mentioned problems, it is obvious that an innovation is needed in the art that will facilitate production of daily single-dose formulations with appropriate release profile comprising metformin and dapagliflozin.

### Background and Description of the Invention

A bilayer tablet formulation comprising an immediate release layer, wherein the said layer contains metformin or a pharmaceutically acceptable salt thereof and dapagliflozin or a pharmaceutically acceptable salt thereof as well as an extended release layer, wherein the said layer contains metformin or a pharmaceutically acceptable salt thereof and, one or more pharmaceutically acceptable excipients.

The object of the present invention is to provide an improved bilayer tablet formulation comprising dapagliflozin or a pharmaceutically acceptable salt thereof combined with metformin or a pharmaceutically acceptable salt thereof, to be used in the treatment of type 2 diabetes, using suitable excipients.

The object of the present invention is to provide a bilayer tablet optionally comprising a film coating with extended release formulations of metformin as well as immediate release formulations of dapagliflozin and metformin.

The object of the present invention is to combine metformin and dapagliflozin in a single dosage form while maintaining stability and improving the dissolution profile thanks to the said bilayer form.

Surprisingly, it has been observed that the dissolution profile was improved with the presence of metformin in both immediate release and extended release layers.

It has been surprisingly found that, according to an embodiment of the present invention, the distribution of metformin active ingredient in the total formulation at certain proportions between the layers not only improved the dissolution profile but also promoted the compressibility of the bilayer tablet.

It has been surprisingly found that, according to an embodiment of the present invention, the use of metformin active ingredient in the immediate release layer at 30 to 85% by weight, preferably 40 to 80%, more preferably 50 to 70% in total formulation in the immediate release layer improved the dissolution profile, reversed the effect of the diffusion barrier formed by the extended release layer in preventing reaching the desired efficacy and facilitated the compressibility of the bilayer tablet.

It has been surprisingly found that, according to an embodiment of the present invention, the use of metformin active ingredient in the extended release layer at 40 to 95% by weight, preferably 50 to 90%, more preferably 60 to 85% in total formulation in the extended release layer improved the dissolution profile and facilitated the compressibility of the bilayer tablet.

According to an embodiment of the present invention, the immediate release layer (first layer) comprise at least one excipient selected from fillers, binders, disintegrants, glidants, lubricants, color agents or mixtures thereof.

According to an embodiment of the present invention, the extended release layer (second layer) comprise at least one excipient selected from fillers, binders, disintegrants, glidants, lubricants or mixtures thereof.

According to an embodiment of the present invention, the amount of filler in the first layer is 10 to 50% by weight, preferably 15 to 40%, more preferably 20 to 35% by weight in the total composition in the first layer.

According to an embodiment of the present invention, the amount of filler in the second layer is 5 to 30% by weight, preferably 8 to 25%, more preferably 10 to 20% by weight in the total composition in the second layer.

It has been surprisingly found that, according to an embodiment of the present invention, the use of metformin with low compressibility rate to the filler in the ratio of 0,6 - 8,5, preferably 1 - 5,5, more preferably 1,4 - 3,5 in the immediate release layer provides more stable bilayer tablet formulation of dapagliflozin and metformin.

According to one embodiment of the present invention, said fillers comprise at least one of lactose, microcrystalline cellulose, starch, mannitol, calcium hydrogen phosphate dihydrate, dicalcium hydrogen phosphate anhydrate, calcium phosphate trihydrate, silicon dioxide and glucose or mixtures thereof. Microcrystalline cellulose is preferably used in the first layer and in the second layer of the invention.

It has been surprisingly found that, according to an embodiment of the present invention, the use of metformin with low compressibility rate to the filler in the ratio of 1,3 - 19, preferably 2 - 11,25, more preferably 3 - 8,5 in the extended release layer provides more stable bilayer tablet formulation of dapagliflozin and metformin.

According to an embodiment of the present invention, the amount of binder in the first layer is 0,1 to 10% by weight, preferably 1 to 7%, more preferably 3 to 5% by weight in the total composition in the first layer.

According to an embodiment of the present invention, the amount of binder in the second layer is 0,1 to 20% by weight, preferably 1 to 15%, more preferably 3 to 12% by weight in the total composition in the second layer.

According to one embodiment of the present invention, said binders comprise at least one of polyvinylpyrrolidone (povidone), hydroxypropylmethylcellulose, hydroxypropylcellulose, carboxymethylcellulose, methyl cellulose, ethyl cellulose, poliox, xantum gum and gelatin, or mixtures thereof. Preferably, polyvinylprolidone is used in the first layer, hydroxypropylmethylcellulose or carboxymethylcellulose or polyox or xanthan gum in the second layer.

According to an embodiment of the present invention, the amount of disintegrant in the first layer is 0,1 to 10% by weight, preferably 1 to 7%, more preferably 3 to 5% by weight in the total composition in the first layer.

According to an embodiment of the present invention, the amount of disintegrant in the second layer is 0,1 to 10% by weight, preferably 1 to 7%, more preferably 3 to 5% by weight in the total composition in the second layer.

According to one embodiment of the present invention, said disintegrants comprise at least one of sodium starch glycollate, croscarmellose sodium, crospovidone, sodium alginate, gums, starch and magnesium aluminum silicate, or a mixture thereof. Preferably, croscarmellose sodium is used in the first layer and in the second layer of the invention.

It has been surprisingly found that, according to an embodiment of the present invention, the use of dapagliflozin with low dissolution to the disintegrant in the ratio of 0,01 - 10, preferably 0,1 - 7, more preferably 0,5 - 1,6 in the immediate release layer provides bilayer tablet formulation of dapagliflozin and metformin with good dissolution and solubility, thus high bioavailability

According to an embodiment of the present invention, the amount of glidant in the first layer is 0,01 to 5% by weight, preferably 0,05 to 3%, more preferably 0,1 to 2% by weight in the total composition in the first layer.

According to an embodiment of the present invention, the amount of glidant in the second layer is 0,01 to 5% by weight, preferably 0,05 to 3%, more preferably 0,1 to 2% by weight in the total composition in the second layer.

According to one embodiment of the present invention, said glidants comprise at least one of colloidal silicon dioxide, talc, aluminum silicate and magnesium silicate, or a mixture thereof. Colloidal silicon dioxide is preferably used in the first layer and in the second layer of the invention.

According to an embodiment of the present invention, the amount of lubricant in the first layer is 0,01 to 5% by weight, preferably 0,05 to 3%, more preferably 0,1 to 2% by weight in the total composition in the first layer.

According to an embodiment of the present invention, the amount of lubricant in the second layer is 0,01 to 5% by weight, preferably 0,05 to 3%, more preferably 0,1 to 2% by weight in the total composition in the second layer.

According to an embodiment of the present invention, said lubricant for the first layer and the second layer is magnesium stearate.

According to an embodiment of the present invention, the amount of coloring agent in the first layer is 0,01 to 5% by weight, preferably 0,05 to 3%, more preferably 0,1 to 2% by weight in the total composition in the first layer.

According to an embodiment of the present invention, said coloring agent for the first layer is iron oxide.

The present invention provides bilayer tablets comprising an immediate release formulation of dapagliflozin and metformin, wherein the first layer comprises dapagliflozin or a pharmaceutically acceptable salt thereof and metformin or a pharmaceutically acceptable salt thereof as active ingredients, microcrystalline cellulose as a filler, polyvinylprolidone as binder, croscarmellose sodium as disintegrant, colloidal silicon dioxide as glidant, magnesium stearate as lubricant and iron oxide as coloring agent as well as an extended release formulation, wherein the second layer comprises metformin or a pharmaceutically acceptable salt thereof as active ingredient, microcrystalline cellulose as filler, hydroxypropylmethylcellulose or carboxymethylcellulose or polyox or xanthan gum as binder, croscarmellose sodium as disintegrant, colloidal silicon dioxide as glidant, magnesium stearate as lubricant and optionally a film coating for both layers.

An embodiment of the present invention is a method for preparing a bilayer tablet formulation, wherein
**1.** the immediate release layer is prepared by:
   a. Dissolving polyvinylprolidone in alcohol and adding iron oxide and mixing
   b. Granulating, drying and sieving of dapagliflozin, metformin and microcrystalline cellulose mixture with the obtained solution
   c. Adding croscarmellose sodium, colloidal silicon dioxide, magnesium stearate and obtaining a homogeneous powder mixture
**2.** the extended release layer is prepared by:
   a. Granulating metformin, microcrystalline cellulose, croscarmellose sodium, hydroxypropylmethyl cellulose or carboxymethylcellulose sodium or polyox or xanthan gum with alcohol and drying and sieving
   b. Adding colloidal silicon dioxide to the mixture and obtaining a homogeneous powder mixture
**3.** Compressing the immediate release layer and extended release layer into a bilayer tablet
**4.** Film coating with moisture-barrier coating

The present invention is described in more detail by the following examples. The example is not to limit the scope of the invention, but should be considered in the light of the details described above.

### Example 1:

| **Active ingredient and excipient** | **Ingredients in one layer (%) (by weight)** |
|---|---|
| **The immediate release layer (first layer)** | |
| Dapagliflozin | 0,1 - 10% |
| Metformin | 30 - 85% |
| Microcrystalline cellulose | 10 - 50% |
| Polyvinylpyrrolidone | 0,1 - 10% |
| Croscarmellose sodium | 0,1 - 10% |
| Colloidal silicon dioxide | 0,01 - 5% |
| Magnesium stearate | 0,01 - 5% |
| Iron oxide | 0,01 - 5% |

| **The immediate release layer (total)** | |
|---|---|
| **The extended release layer (second layer)** | |
| Metformin | 40 - 95% |
| Microcrystalline cellulose | 5 - 30% |
| Hydroxypropylmethylcellulose or carboxymethylcellulose sodium or polyox or xanthan gum | 0,1 - 20% |
| Croscarmellose sodium | 0,1 - 10% |
| Colloidal silicon dioxide | 0,01 - 5% |
| Magnesium stearate | 0,01 - 5% |

| **The extended release layer (total)** | |
|---|---|
| **Film-coating solution** | |
| Moisture-barrier coating | 1 - 10% |
| **The weight of the film tablet (total)** | 100% |

The above-mentioned pharmaceutical formulation is prepared as follows:
**The extended release layer:** Polyvinylprolidone is dissolved in alcohol, then iron oxide is added and mixed. Dapagliflozin, metformin, microcrystalline cellulose mixture is granulated with this solution, dried and sieved. A homogeneous powder mixture is obtained by adding the remaining excipients.

**The extended release layer:** Metformin, microcrystalline cellulose, hydroxypropylmethylcellulose or carboxymethylcellulose sodium or polyox or xanthan gum, croscarmellose sodium alcohol are granulated, dried and sieved. A homogeneous powder mixture is obtained by adding the remaining excipients.

**Tablet compression:** The immediate release layer and extended release layer are compressed to form a bilayer tablet.

**Film coating:** Film coating with moisture-barrier coating.

### Example 2:

| **Active ingredient and excipient** | **Ingredients in one layer (%) (by weight)** |
|---|---|
| **The immediate release layer (first layer)** | |
| Dapagliflozin | 1 - 7% |
| Metformin | 40 - 80% |
| Microcrystalline cellulose | 15 - 40% |
| Polyvinylpyrrolidone | 1 - 7% |
| Croscarmellose sodium | 1 - 7% |
| Colloidal silicon dioxide | 0,05 - 3% |
| Magnesium stearate | 0,05 - 3% |
| Iron oxide | 0,05 - 3% |

| **The immediate release layer (total)** | |
|---|---|
| **The extended release layer (second layer)** | |
| Metformin | 50 - 90% |
| Microcrystalline cellulose | 8 - 25% |
| Hydroxypropylmethylcellulose or carboxymethylcellulose sodium or polyox or xanthan gum | 1 - 15% |
| Croscarmellose sodium | 1 - 7% |
| Colloidal silicon dioxide | 0,05 - 3% |
| Magnesium stearate | 0,05 - 3% |

| **The extended release layer (total)** | |
|---|---|
| **Film-coating solution** | |
| Moisture-barrier coating | 2 - 8% |
| **The weight of the film tablet (total)** | 100% |

The above-mentioned pharmaceutical formulation is prepared as follows:
**The extended release layer:** Polyvinylprolidone is dissolved in alcohol, then iron oxide is added and mixed. Dapagliflozin, metformin, microcrystalline cellulose mixture is granulated with this solution, dried and sieved. A homogeneous powder mixture is obtained by adding the remaining excipients.

**The extended release layer:** Metformin, microcrystalline cellulose, hydroxypropylmethylcellulose or carboxymethylcellulose sodium or polyox or xanthan gum, croscarmellose sodium alcohol are granulated, dried and sieved. A homogeneous powder mixture is obtained by adding the remaining excipients.

**Tablet compression:** The immediate release layer and extended release layer are compressed to form a bilayer tablet.

**Film coating:** Film coating with moisture-barrier coating.

### Example 3:

| **Active ingredient and excipient** | **Ingredients in one layer (%) (by weight)** |
|---|---|
| **The immediate release layer (first layer)** | |
| Dapagliflozin | 3 - 5% |
| Metformin | 50 - 70% |
| Microcrystalline cellulose | 20 - 35% |
| Polyvinylpyrrolidone | 3 - 5% |
| Croscarmellose sodium | 3 - 5% |
| Colloidal silicon dioxide | 0,1 - 2% |
| Magnesium stearate | 0,1 - 2% |
| Iron oxide | 0,1 - 2% |
| **The immediate release layer (total)** | 100% |

| **The extended release layer (second layer)** | |
|---|---|
| Metformin | 60 - 85% |
| Microcrystalline cellulose | 10 - 20% |
| Hydroxypropylmethylcellulose or carboxymethylcellulose sodium or polyox or xanthan gum | 3 - 12% |
| Croscarmellose sodium | 3 - 5% |
| Colloidal silicon dioxide | 0,1 - 2% |
| Magnesium stearate | 0,1 - 2% |
| **The extended release layer (total)** | 100% |

| **Film-coating solution** | |
|---|---|
| Moisture-barrier coating | 5 - 6% |
| **The weight of the film tablet (total)** | 100% |

The above-mentioned pharmaceutical formulation is prepared as follows:
**The immediate release layer:** Polyvinylprolidone is dissolved in alcohol, then iron oxide is added and mixed. Dapagliflozin, metformin, microcrystalline cellulose mixture is granulated with this solution, dried and sieved. A homogeneous powder mixture is obtained by adding the remaining excipients.

**The extended release layer:** Metformin, microcrystalline cellulose, hydroxypropylmethylcellulose or carboxymethylcellulose sodium or polyox or xanthan gum, croscarmellose sodium alcohol are granulated, dried and sieved. A homogeneous powder mixture is obtained by adding the remaining excipients.

**Tablet compression:** The immediate release layer and extended release layer are compressed to form a bilayer tablet.

**Film coating:** Film coating with moisture-barrier coating.

With the invention, surprisingly, a bilayer tablet formulation of metformin and dapagliflozin with the desired release profile is obtained. The immediate release and extended release layers in the bilayer tablet have been used in such proportions, so that a formulation with suitable and desired properties could be obtained which provides up to 24 hours of release. The start of treatment is achieved at the desired level depending on the dissolution rate of the immediate release layer.

## Claims

1. A bilayer tablet formulation comprising an immediate release layer, wherein the said layer contains metformin or a pharmaceutically acceptable salt thereof and dapagliflozin or a pharmaceutically acceptable salt thereof as well as an extended release layer, wherein the said layer contains metformin or a pharmaceutically acceptable salt thereof and, one or more pharmaceutically acceptable excipients.

2. The bilayer tablet composition according to Claim 1, wherein metformin or a pharmaceutically active salt thereof is present in the range of 30 to 85% by weight, preferably 40 to 80%, more preferably 50 to 70% in total formulation in the immediate release layer and metformin active ingredient in the extended release layer is present in the range of 40 to 95% by weight, preferably 50 to 90%, more preferably 60 to 85% in total formulation in the extended release layer.

3. The bilayer tablet composition according to Claim 1 or 2, wherein more than one pharmaceutically acceptable excipient is selected from the group comprising fillers, binders, dispersants, glidants, lubricants, color agents or mixtures thereof in the immediate release and extended release layer.

4. The bilayer tablet composition according to Claim 3, wherein the amount of filler in the immediate release layer is 10 to 50% by weight, preferably 15 to 40%, more preferably 20 to 35% by weight in the total composition in the immediate release layer and the amount of filler in the extended release layer is 5 to 30% by weight, preferably 8 to 25%, more preferably 10 to 20% by weight in the total composition in the extended release layer.

5. The bilayer tablet composition according to Claim 3, wherein the amount of metformin active ingredient in the immediate release layer to the filler in the immediate release layer is in the range of 0,6 - 8,5, preferably 1 - 5,5, more preferably 1,4 - 3,5 and the amount of metformin active ingredient in the extended release layer to the filler in the extended release layer is in the range of 1,3 - 19, preferably 2 - 11,25, more preferably 3 - 8,5.

6. The bilayer tablet composition according to the preceding claims, wherein the composition comprise at least one of lactose, microcrystalline cellulose, starch, mannitol, calcium hydrogen phosphate dihydrate, dicalcium hydrogen phosphate anhydrate, calcium phosphate trihydrate, silicon dioxide and glucose or mixtures thereof as a filler and preferably the filler is microcrystalline cellulose.

7. The bilayer tablet composition according to Claim 3, wherein the amount of binder in the immediate release layer is 0,1 to 10% by weight, preferably 1 to 7%, more preferably 3 to 5% by weight in the total composition in the immediate release layer and the amount of binder in the extended release layer is 0,1 to 20% by weight, preferably 1 to 15% and more preferably 3 to 12% by weight in the total composition in the extended release layer.

8. The bilayer tablet composition according to the preceding claims, wherein the composition comprises at least one of polyvinylpyrrolidone (povidone), hydroxypropylmethylcellulose, hydroxypropylcellulose, carboxymethylcellulose, methyl cellulose, ethyl cellulose, poliox, xantum gum and gelatin, or mixtures thereof as a binder and preferably the binder is polyvinylprolidone in the immediate release layer and hydroxypropylmethylcellulose or carboxymethylcellulose or polyox or xanthan gum in the extended release layer.

9. The bilayer tablet composition according to Claim 3, wherein the amount of disintegrant in the immediate release layer is 0,1 to 10% by weight, preferably 1 to 7%, more preferably 3 to 5% by weight in the total composition in the immediate release layer and the amount of disintegrant in the extended release layer is 0,1 to 10% by weight, preferably 1 to 7% and more preferably 3 to 5% by weight in the total composition in the extended release layer.

10. The bilayer tablet composition according to Claim 3, wherein the amount of dapagliflozin active ingredient in the immediate release layer to the disintegrant is in the range of 0,01 - 10, preferably 0,1 - 7, more preferably 0,5 - 1,6.

11. The bilayer tablet composition according to Claim 3, 9 and 10, wherein the composition comprises at least one of sodium starch glycollate, croscarmellose sodium, crospovidone, sodium alginate, gums, starch and magnesium aluminum silicate, or a mixture thereof as a disintegrant and the disintegrant is preferably croscarmellose sodium.

12. The bilayer tablet composition according to Claim 3, wherein the amount of glidant in the immediate release layer is 0,01 to 5% by weight, preferably 0,05 to 3%, more preferably 0,1 to 2% by weight in the total composition in the immediate release layer and the amount of glidant in the extended release layer is 0,01 to 5% by weight, preferably 0,05 to 3%, more preferably 0,1 to 2% by weight in the total composition in the extended release layer.

13. The bilayer tablet composition according to the preceding claims, wherein the composition comprises the following;
(i) the immediate release layer:
a. 0,1 to 10% by weight of dapagliflozin or a pharmaceutically acceptable salt thereof
b. 30 - 85% by weight of metformin or a pharmaceutically acceptable salt thereof
c. 10 - 50% by weight of microcrystalline cellulose
d. 0,1 - 10% by weight of polyvinylprolidone
e. 0,1 - 10% by weight of croscarmellose sodium
f. 0,01 - 5% by weight of colloidal silicon dioxide
g. 0,01 - 5% by weight of magnesium stearate
h. 0,01 - 5% by weight of iron oxide
(ii) The extended release layer:
a. 40 - 95% by weight of metformin
b. 5 - 30% by weight of microcrystalline cellulose
c. 0,1 - 20% by weight of hydroxypropylmethylcellulose or carboxymethylcellulose sodium or polyox or xanthan gum
d. 0,1 - 10% by weight of croscarmellose sodium
e. 0,01 - 5% by weight colloidal silicon dioxide
f. 0,01 - 5% by weight of magnesium stearate
(iii) Film-coating solution:
1-10% by weight of moisture-barrier coating

14. The bilayer tablet composition according to the preceding claims, wherein the composition comprises the following production method;
(i) the immediate release layer:
a. Dissolving polyvinylprolidone in alcohol and adding iron oxide and mixing
b. Granulating, drying and sieving of dapagliflozin, metformin and microcrystalline cellulose mixture with the obtained solution
c. Adding croscarmellose sodium, colloidal silicon dioxide, magnesium stearate and obtaining a homogeneous powder mixture
(ii) The extended release layer:
a. Granulating metformin, microcrystalline cellulose, croscarmellose sodium, hydroxypropylmethyl cellulose or carboxymethylcellulose sodium or polyox or xanthan gum with alcohol and drying and sieving
b. Adding colloidal silicon dioxide to the mixture and obtaining a homogeneous powder mixture
(iii) Compression of immediate release layer and extended release layer to form a bilayer tablet
(iv) Film coating of the compressed tablet with moisture-barrier coating.

## Patentansprüche

1. Eine zweischichtige Tablettenformulierung, umfassend eine sofort freisetzende Schicht, wobei diese Schicht Metformin oder ein pharmazeutisch akzeptables Salz davon und Dapagliflozin oder ein pharmazeutisch akzeptables Salz davon enthält, sowie eine verzögert freisetzende Schicht, wobei die Schicht Metformin oder ein pharmazeutisch akzeptables Salz davon und einen oder mehrere pharmazeutisch akzeptable Hilfsstoffe enthält.

2. Zweischichtige Tablettenzusammensetzung nach Anspruch 1, wobei Metformin oder ein pharmazeutisch wirksames Salz davon in einem Bereich von 30 bis 85 Gew.-%, vorzugsweise 40 bis 80 Gew.-%, noch bevorzugter 50 bis 70 % in der Gesamtformulierung in der Schicht mit sofortiger Freisetzung enthalten ist und der Wirkstoff Metformin in der Schicht mit verlängerter Freisetzung im Bereich von 40 bis 95 Gew.-%, vorzugsweise 50 bis 90 %, noch bevorzugter 60 bis 85 % in der Gesamtformulierung in der Schicht mit verlängerter Freisetzung enthalten ist.

3. Zweischichtige Tablettenzusammensetzung nach Anspruch 1 oder 2, wobei mehr als ein pharmazeutisch akzeptabler Hilfsstoff aus der Gruppe ausgewählt ist, die Füllstoffen, Bindemitteln, Dispergiermitteln, Gleitmitteln, Schmiermitteln, Farbstoffen oder Mischungen davon in der sofort freisetzenden und der verzögerten Freisetzungsschicht umfasst.

4. Zweischichtige Tablettenzusammensetzung nach Anspruch 3, wobei die Menge an Füllstoff in der sofort freisetzenden Schicht 10 bis 50 Gew.-%, vorzugsweise 15 bis 40 Gew.-%, noch bevorzugter 20 bis 35 Gew.-% der Gesamtzusammensetzung in der Schicht mit sofortiger Freisetzung beträgt und die Menge an Füllstoff in der Schicht mit verzögerter Freisetzung 5 bis 30 Gew.-%, vorzugsweise 8 bis 25 Gew.-%, noch bevorzugter 10 bis 20 Gew.-% der Gesamtzusammensetzung in der Schicht mit verzögerter Freisetzung beträgt.

5. Zweischichtige Tablettenzusammensetzung nach Anspruch 3, wobei die Menge an Metformin-Wirkstoff in der sofort freisetzenden Schicht im Verhältnis zum Füllstoff in der sofort freisetzenden Schicht im Bereich von 0,6 - 8,5, vorzugsweise 1 - 5,5, noch bevorzugter 1,4 - 3,5, und die Menge an Metformin-Wirkstoff in der Schicht mit verzögerter Freisetzung im Verhältnis zum Füllstoff in der Schicht mit verzögerter Freisetzung im Bereich von 1,3 - 19, vorzugsweise 2 - 11,25, noch bevorzugter 3 - 8,5 liegt.

6. Zweischichtige Tablettenzusammensetzung nach den vorstehenden Ansprüchen, wobei die Zusammensetzung mindestens eines von Laktose, mikrokristalline Cellulose, Stärke, Mannitol, Calciumhydrogenphosphat-Dihydrat, Dicalciumhydrogenphosphat-Anhydrat, Calciumphosphat-Trihydrat, Siliciumdioxid und Glucose oder Mischungen davon als Füllstoff umfasst und vorzugsweise der Füllstoff mikrokristalline Cellulose ist.

7. Zweischichtige Tablettenzusammensetzung nach Anspruch 3, wobei die Menge an Bindemittel in der sofort freisetzenden Schicht 0,1 bis 10 Gew.-%, vorzugsweise 1 bis 7 Gew.-%, noch bevorzugter 3 bis 5 Gew.-% der Gesamtzusammensetzung in der sofort freisetzenden Schicht beträgt und die Menge an Bindemittel in der verzögerten Freisetzungsschicht 0,1 bis 20 Gew.-%, vorzugsweise 1 bis 15 Gew.-% und noch bevorzugter 3 bis 12 Gew.-% der Gesamtzusammensetzung in der verzögerten Freisetzungsschicht beträgt.

8. Zweischichtige Tablettenzusammensetzung nach den vorstehenden Ansprüchen, wobei die Zusammensetzung mindestens eines der folgenden Bindemittel umfasst, Polyvinylpyrrolidon (Povidon), Hydroxypropylmethylcellulose, Hydroxypropylcellulose, Carboxymethylcellulose, Methylcellulose, Ethylcellulose, Polyox, Xanthan-Gummi und Gelatine oder Mischungen davon, wobei das Bindemittel vorzugsweise Polyvinylpyrrolidon in der sofort freisetzenden Schicht und Hydroxypropylmethylcellulose oder Carboxymethylcellulose oder Polyox oder Xanthan-Gummi in der verzögerte freisetzenden Schicht ist.

9. Zweischichtige Tablettenzusammensetzung nach Anspruch 3, wobei die Menge an Sprengmittel in der sofort freisetzenden Schicht 0,1 bis 10 Gew.-%, vorzugsweise 1 bis 7 Gew.-%, noch bevorzugter 3 bis 5 Gew.-% der Gesamtzusammensetzung in der sofort freisetzenden Schicht beträgt und die Menge an Sprengmittel in der verzögerten Freisetzungsschicht 0,1 bis 10 Gew.-%, vorzugsweise 1 bis 7 Gew.-% und noch bevorzugter 3 bis 5 Gew.-% der Gesamtzusammensetzung in der verzögerten Freisetzungsschicht beträgt.

10. Zweischichtige Tablettenzusammensetzung nach Anspruch 3, wobei die Menge an Dapagliflozin-Wirkstoff in der sofort freisetzenden Schicht im Verhältnis zum Sprengmittel im Bereich von 0,01 - 10, vorzugsweise 0,1 - 7, noch bevorzugter 0,5 - 1,6 liegt.

11. Zweischichtige Tablettenzusammensetzung nach Anspruch 3, 9 und 10, wobei die Zusammensetzung mindestens eines von Natriumstärkeglykolat, Croscarmellose-Natrium, Crospovidon, Natriumalginat, Gummis, Stärke und Magnesiumaluminiumsilikat oder eine Mischung davon als Sprengmittel umfasst und das Sprengmittel vorzugsweise Croscarmellose-Natrium ist.

12. Zweischichtige Tablettenzusammensetzung nach Anspruch 3, wobei die Menge an Gleitmittel in der sofort freisetzenden Schicht 0,01 bis 5 Gew.-%, vorzugsweise 0,05 bis 3 Gew.-%, noch bevorzugter 0,1 bis 2 Gew.-% der Gesamtzusammensetzung in der sofort freisetzenden Schicht beträgt und die Menge an Gleitmittel in der verzögerten Freisetzungsschicht 0,01 bis 5 Gew.-%, vorzugsweise 0,05 bis 3 Gew.-%, noch bevorzugter 0,1 bis 2 Gew.-% der Gesamtzusammensetzung in der verzögerten Freisetzungsschicht beträgt.

13. Zweischichtige Tablettenzusammensetzung gemäß den vorstehenden Ansprüchen, wobei die Zusammensetzung Folgendes umfasst:
(i) die sofort freisetzende Schicht:
a. 0,1 bis 10 Gew.-% Dapagliflozin oder ein pharmazeutisch akzeptables Salz davon
b. 30 - 85 Gew.-% Metformin oder ein pharmazeutisch akzeptables Salz davon
c. 10 - 50 Gew.-% mikrokristalline Cellulose
d. 0,1 - 10 Gew.-% Polyvinylpyrrolidon
e. 0,1 - 10 Gew.-% Croscarmellose-Natrium
f. 0,01 - 5 Gew.-% kolloidales Siliciumdioxid
g. 0,01-5 Gew.-% Magnesiumstearat
h. 0,01-5 Gew.-% Eisenoxid
(ii) die verzögert freisetzende Schicht:
a. 40-95 Gew.-% Metformin
b. 5-30 Gew.-% mikrokristalline Cellulose
c. 0,1-20 Gew.-% Hydroxypropylmethylcellulose oder Carboxymethylcellulose-Natrium oder Polyox oder Xanthan-Gummi
d. 0,1-10 Gew.-% Croscarmellose-Natrium
e. 0,01-5 Gew.-% kolloidales Siliciumdioxid
f. 0,01-5 Gew.-% Magnesiumstearat
(iii) Filmüberzugsmittel:
1-10 Gew.-% Feuchtigkeitsbarrierebeschichtung

14. Zweischichtige Tablettenzusammensetzung gemäß den vorstehenden Ansprüchen, wobei die Zusammensetzung das folgende Herstellungsverfahren umfasst:
(i) die sofort freisetzende Schicht:
a. Auflösen von Polyvinylpyrrolidon in Alkohol und Hinzufügen von Eisenoxid und Mischen
b. Granulieren, Trocknen und Sieben einer Mischung aus Dapagliflozin, Metformin und mikrokristalline Cellulose mit der erhaltenen Lösung
c. Zugabe von Croscarmellose-Natrium, kolloidalem Siliciumdioxid, Magnesiumstearat und Erhalt einer homogenen Pulvermischung
(ii) Die verzögert freisetzende Schicht:
a. Granulieren von Metformin, mikrokristalline Cellulose, Croscarmellose-Natrium, Hydroxypropylmethylcellulose oder Carboxymethylcellulose-Natrium oder Polyox oder Xanthan-Gummi mit Alkohol und Trocknen und Sieben
b. Hinzufügen von kolloidalem Siliciumdioxid zu der Mischung und Erhalten einer homogenen Pulvermischung
(iii) Verpressen der sofort freisetzenden Schicht und der verzögert freisetzenden Schicht zu einer zweischichtigen Tablette
(iv) Filmbeschichtung der gepressten Tablette mit einer Feuchtigkeitsbarrierebeschichtung.

## Revendications

1. - Formulation de comprimé bicouche comprenant une couche à libération immédiate, ladite couche contenant de la metformine ou un sel pharmaceutiquement acceptable de celle-ci et de la dapagliflozine ou un sel pharmaceutiquement acceptable de celle-ci, ainsi qu'une couche à libération prolongée, ladite couche contenant de la metformine ou un sel pharmaceutiquement acceptable de celle-ci, et un ou plusieurs excipients pharmaceutiquement acceptables.

2. - Composition de comprimé bicouche selon la revendication 1, dans laquelle la metformine ou un sel pharmaceutiquement actif de celle-ci est présent dans la plage de 30 à 85% en poids, de préférence de 40 à 80%, de façon davantage préférée de 50 à 70% dans la formulation totale dans la couche à libération immédiate et le principe actif de metformine dans la couche à libération prolongée est présent dans la plage de 40 à 95% en poids, de préférence de 50 à 90%, de façon davantage préférée de 60 à 85% dans la formulation totale dans la couche à libération prolongée.

3. - Composition de comprimé bicouche selon la revendication 1 ou 2, dans laquelle le ou les excipients pharmaceutiquement acceptables est/sont choisi(s) dans le groupe comprenant les charges, les liants, les dispersants, les agents de glissement, les lubrifiants, les agents colorants ou leurs mélanges dans la couche à libération immédiate et la couche à libération prolongée.

4. - Composition de comprimé bicouche selon la revendication 3, dans laquelle la quantité de charge dans la couche à libération immédiate est de 10 à 50 % en poids, de préférence de 15 à 40 %, de façon davantage préférée de 20 à 35 % en poids dans la composition totale dans la couche à libération immédiate et la quantité de charge dans la couche à libération prolongée est de 5 à 30 % en poids, de préférence de 8 à 25 %, de façon davantage préférée de 10 à 20 % en poids dans la composition totale dans la couche à libération prolongée.

5. - Composition de comprimé bicouche selon la revendication 3, dans laquelle la quantité de principe actif de metformine dans la couche à libération immédiate par rapport à la charge dans la couche à libération immédiate est dans la plage de 0,6 à 8,5, de préférence de 1 à 5,5, de façon davantage préférée de 1,4 à 3,5 et la quantité de principe actif de metformine dans la couche à libération prolongée par rapport à la charge dans la couche à libération prolongée est dans la plage de 1,3 à 19, de préférence de 2 à 11,25, de façon davantage préférée de 3 à 8,5.

6. - Composition de comprimé bicouche selon les revendications précédentes, dans laquelle la composition comprend au moins l'un parmi le lactose, la cellulose microcristalline, l'amidon, le mannitol, le dihydrate d'hydrogéno phosphate de calcium, l'anhydrate d'hydrogéno phosphate bicalcique, le trihydrate de phosphate de calcium, le dioxyde de silicium et le glucose ou leurs mélanges en tant que charge et, de préférence, la charge est la cellulose microcristalline.

7. - Composition de comprimé bicouche selon la revendication 3, dans laquelle la quantité de liant dans la couche à libération immédiate est de 0,1 à 10% en poids, de préférence de 1 à 7%, de façon davantage préférée de 3 à 5% en poids dans la composition totale dans la couche à libération immédiate et la quantité de liant dans la couche à libération prolongée est de 0,1 à 20% en poids, de préférence de 1 à 15% et de façon davantage préférée de 3 à 12% en poids dans la composition totale de la couche à libération prolongée.

8. - Composition de comprimé bicouche selon les revendications précédentes, dans laquelle la composition comprend au moins l'un parmi la polyvinylpyrrolidone (povidone), l'hydroxypropylméthylcellulose, l'hydroxypropylcellulose, la carboxyméthylcellulose, la méthyl cellulose, l'éthyl cellulose, le poliox, la gomme de xanthane et la gélatine, ou leurs mélanges, comme liant, et de préférence, le liant est la polyvinylpyrrolidone dans la couche à libération immédiate et l'hydroxypropylméthylcellulose ou la carboxyméthylcellulose ou le polyox ou la gomme de xanthane dans la couche à libération prolongée.

9. - Composition de comprimé bicouche selon la revendication 3, dans laquelle la quantité de désintégrant dans la couche à libération immédiate est de 0,1 à 10% en poids, de préférence de 1 à 7%, de façon davantage préférée de 3 à 5% en poids dans la composition totale dans la couche à libération immédiate et la quantité de désintégrant dans la couche à libération prolongée est de 0,1 à 10% en poids, de préférence de 1 à 7% et de façon davantage préférée de 3 à 5% en poids dans la composition totale dans la couche à libération prolongée.

10. - Composition de comprimé bicouche selon la revendication 3, dans laquelle la quantité de principe actif de dapagliflozine dans la couche à libération immédiate par rapport au désintégrant est dans la plage de 0,01 à 10, de préférence de 0,1 à 7, de façon davantage préférée de 0,5 à 1,6.

11. - Composition de comprimé bicouche selon l'une des revendications 3, 9 et 10, dans laquelle la composition comprend au moins l'un parmi le glycollate d'amidon sodique, la croscarmellose sodique, la crospovidone, l'alginate de sodium, les gommes, l'amidon et le silicate d'aluminium et de magnésium, ou un mélange de ceux-ci, en tant que désintégrant, et le désintégrant est, de préférence, la croscarmellose sodique.

12. - Composition de comprimé bicouche selon la revendication 3, dans laquelle la quantité d'agent de glissement dans la couche à libération immédiate est de 0,01 à 5% en poids, de préférence de 0,05 à 3%, de façon davantage préférée de 0,1 à 2% en poids dans la composition totale dans la couche à libération immédiate et la quantité d'agent de glissement dans la couche à libération prolongée est de 0,01 à 5% en poids, de préférence de 0,05 à 3%, de façon davantage préférée de 0,1 à 2% en poids dans la composition totale dans la couche à libération prolongée.

13. - Composition de comprimé bicouche selon les revendications précédentes, dans laquelle la composition comprend ce qui suit :
(i) la couche à libération immédiate :
a. 0,1 à 10 % en poids de dapagliflozine ou d'un sel pharmaceutiquement acceptable de celle-ci
b. 30 à 85 % en poids de metformine ou d'un sel pharmaceutiquement acceptable de celle-ci
c. 10 à 50 % en poids de cellulose microcristalline
d. 0,1 à 10 % en poids de polyvinylpyrrolidone
e. 0,1 à 10 % en poids de croscarmellose sodique
f. 0,01 à 5 % en poids de dioxyde de silicium colloïdal
g. 0,01 à 5 % en poids de stéarate de magnésium
h. 0,01 à 5 % en poids d'oxyde de fer
(ii) la couche à libération prolongée :
a. 40 à 95 % en poids de metformine
b. 5 à 30 % en poids de cellulose microcristalline
c. 0,1 à 20 % en poids d'hydroxypropylméthylcellulose ou de carboxyméthylcellulose sodique ou de polyox ou de gomme de xanthane
d. 0,1 à 10 % en poids de croscarmellose sodique
e. 0,01 à 5 % en poids de dioxyde de silicium colloïdal
f. 0,01 à 5 % en poids de stéarate de magnésium
(iii) solution de pelliculage :
1 à 10% en poids d'enrobage formant une barrière contre l'humidité.

14. - Composition de comprimé bicouche selon les revendications précédentes, dans laquelle la composition comprend la méthode de production suivante :
(i) la couche à libération immédiate :
a. Dissolution de la polyvinylpyrrolidone dans de l'alcool et addition d'oxyde de fer et mélange ;
b. Granulation, séchage et tamisage du mélange de la dapagliflozine, de la metformine et de la cellulose microcristalline avec la solution obtenue
c. Addition de croscarmellose sodique, de dioxyde de silicium colloïdal, de stéarate de magnésium et obtention d'un mélange pulvérulent homogène
(ii) la couche à libération prolongée :
a. Granulation de la metformine, de la cellulose microcristalline, de la croscarmellose sodique, de l'hydroxypropylméthyl cellulose ou de la carboxyméthyl cellulose sodique ou du polyox ou de la gomme de xanthane avec de l'alcool, et séchage et tamisage
b. Addition de dioxyde de silicium colloïdal au mélange et obtention d'un mélange pulvérulent homogène
(iii) Compression de la couche à libération immédiate et de la couche à libération prolongée pour former un comprimé bicouche
(iv) Pelliculage du comprimé ayant subi la compression avec le revêtement formant une barrière contre l'humidité.
